# EUROPEAN PATENT APPLICATION

(11) **EP 2 444 388 A1**
(43) Date of publication of application: **25.04.2012**
(21) Application number: 10382263.1
(22) Date of filing: 06.10.2010
(51) Int. Cl.: C07C 67/03, C07C 67/08, C10M 105/38, C10M 105/42, C11C 3/00, C07C 69/67, C07C 67/26

(54) **Asymmetric esters of fatty acids useful as lubricants**

(71) Applicant: Industrial Quimica Lasem, S.A., 08297 Castellgalí (ES)
(72) Inventor: García Colomer, Albert, 17300, Blanes (ES); Granero Torne, Francesc, 08224, Terrassa (ES); Beltran Giralt, Lluís, 08041, Barcelona (ES); Planas Sauter, Antoni, 08198, Sant Cugat del Vallès (ES)
(74) Representative: Jané Bonet, Montserrat

(57) **Abstract**

Composition comprising at least one ester of one or more saturated C₁₈ fatty acids and an alcohol, wherein the fatty acids have between 1 and 3 pairs of vicinal ester groups of formula R₁COO-, the two vicinal ester groups in each pair being different, R₁ is a radical independently selected from linear or branched (C₁-C₁₈) alkyl; and the alcohol is selected from the group consisting of a (C₁-C₁₅) linear or branched monohydric alcohol and a (C₂-C₆) polyhydric alcohol having between 2 and 4 hydroxyl groups obtainable by a process comprising: a) epoxidizing a product comprising at least an ester of an unsaturated C₁₈ fatty acid and of an alcohol; followed by ring opening of the epoxide and b) reacting the compound obtained in step a) with a (C₁-C₁₈) linear or branched carboxylic acid different from the one used in step a). The composition obtainable by the process of the invention is useful as lubricant base stock oil or as an additive to modulate the viscosity and/or the pour point of lubricant compounds.

## Description

The present invention relates to new asymmetric esters of fatty acids, their preparation process, as well as their use as lubricants or as additives for lubricants.

### BACKGROUND ART

Vegetal oils have been used extensively as lubricants being preferred to their petroleum-based counterparts because of its biodegradability. However, vegetal oils show several limitations when compared with petroleum-based lubricants. Petroleum-based lubricants maintain their operating properties at high temperatures (chemical and oxidative stability) while showing good operating properties in cold (pour point and cloud point), i.e. being liquid at room temperature. Those properties are linked to the chemical structure of the oils.

Vegetal oils have a high content of esters of unsaturated fatty acids. The steric constraints due to the cis conformation of the unsaturations, i.e. double bonds, of the fatty acids provide excellent properties to vegetal oils at low temperatures, maintaining a low pour point and low viscosity. However, low oxidative stability of unsaturations at high temperature renders vegetable oils unsuitable for most of their target applications in lubrication.

On the other hand, esters of saturated fatty acids show greater oxidation stability at high temperatures but they are solid at temperatures even higher than ambient.

Several processes are known in the art to modify vegetal oils in an effort to provide them with improved thermal and chemical properties and making them suitable for lubrication and other industrial purposes, e.g. as lubricants or compression fluids in hydraulic systems.

On a first stage of modification, fatty acids have been esterified with different alcohols other than glycerine, i.e. pentaerythritol, neopentilglycol, and trimethylolpropane, to obtain ester bonds which are more thermally stable. Although the obtained fatty acid esters show improved thermal properties, the use of unsaturated fatty acids is still preferred in the art to obtain a product with suitable properties at cold temperature. Thus oxidative instability at high temperatures of unsaturations continues to be an issue.

Further attempts to improve the properties of vegetable oils have been done eliminating the unsaturations of the fatty acid esters by introducing substituents in the unsaturation sites. Steric constraints generated by branching on unsaturation sites provide products with improved oxidative stability while retaining good properties at low temperatures.

Hwang et al., "Modification of epoxidized soybean oil for lubricant formulations with improved oxidative stability and low pour point", JAOCS, 2001, vol. 78, n. 12, pp. 1179-1184, discloses a process for eliminating unsaturations of a fatty acid ester which comprises epoxidizing the double bonds of the unsaturations and opening the oxirane ring by the introduction of an alcohol in acid medium and further reaction with a short chain acid anhydride in pyridine to obtain a heavily transesterified product where unsaturations have been substituted by vicinal ether-ester groups. Besides the drawbacks of the use of such dangerous and toxic reagents as pyridine and organic solvents, which make the process troublesome for industrial application, the disclosed process results in a product with ether substitutions, which is prone to forming hydrogen bonds and retaining moisture in the product. In addition, the disclosed process does not retain the primary ester of the fatty acid and the alcohol because of the presence of an alcohol in acidic medium when opening the oxirane ring. The resulting transesterification reaction renders a product which is highly contaminated with the eliminated alcohol of the original fatty acid ester and the partial esters thereof. However, Hwang et al., "Synthetic Lubricant Basestocks from Epoxidized Soybean Oil and Guerbert Alcohols", Industrial Crops and Products, Vol. 23, 311 - 317, 2006 following the same strategy as the process disclosed by Hwang in 2001, minimize the transesterification by adjusting the experimental conditions.

Erhan et al., "Chemical modification of vegetable oils for lubricant base stocks" 2003 Spring Technical Conference of the ASME Internal Combustion Engine Division, American Society of Mechanical Engineers, Salzburg, Austria, pp. 369-380, and Erhan et al. "Lubricant base stock potential of chemically modified vegetable oils" J. Agric. Food Chem., 2008, Vol. 56, issue 19, pp. 8919-8925, disclose processes for the preparation of fatty acid esters, wherein the unsaturations of the fatty acid have been functionalized by symmetric ester groups, i.e. both vicinal ester have the same alkyl chain. In the process disclosed by Erhan et al. in 2003, the oxirane ring is opened by reaction with water to obtain a vicinal diol which is further reacted with an acid anhydride in pyridine. The process disclosed in 2008 directly performs the oxirane ring opening in the presence of an acid anhydride and boron trifluoride etherate. Both processes yield fatty acid esters, wherein the unsaturations of the fatty acid have been functionalized by symmetric ester groups by the reaction with acid anhydride.

Patent application WO2008/079901 discloses a process for the preparation of a fatty acid ester, wherein the unsaturations of the fatty acid have been functionalized by vicinal alcohol and ester groups. The disclosed process comprises the opening of the oxirane ring of the epoxidized fatty acid ester by reaction with a carboxylic acid rendering the vicinal alcohol-ester group. The obtained fatty acid ester, wherein the unsaturations of the fatty acid have been functionalized by vicinal alcohol and ester groups, has a high content of free hydroxyl groups making it prone to the formation of intramolecular and intermolecular hydrogen bonds as well as with water, adding moisture to the oil product.

The presence of moisture is undesirable for products which are expected to show a predictable change of their properties when heating. Groups which are prone to form dipolar interactions such as hydrogen bonds, e.g. ether or hydroxyl groups, both intra or inter molecularly, are undesirable since such interactions change widely with temperature dramatically affecting lubrication and stability properties of the oily products containing them. Such dipolar interactions can also make possible the absorption of moisture, causing the undesirable effect that moisture could evaporate when temperature rise in working conditions, thus changing suddenly the properties of the oily product. Moreover, presence of moisture can cause undesirable ester hydrolysis and metal corrosion.

Thus, there is the need in the art of having at one's disposal a process for modifying fatty acid esters which renders possible to eliminate the unsaturations of the fatty acids maintaining the structure of the primary ester, providing a fine control of the structure and properties of the obtained product, and using safe and readily available reagents.

### SUMMARY OF THE INVENTION

The present inventors have found a process that allows the introduction of asymmetrical vicinal esters in the unsaturation sites of a fatty acid ester, while maintaining the core structure of the starting product, rendering a wide variety of branching degrees and providing a fine control over the properties of the final product.

Unlike known processes for the modification of vegetal oils, the present process allows to obtain different products saturated by asymmetrical vicinal esters which show properties e.g. viscosity, pour point and cloud point, varying in a very wide range. An advantage of the process is that it allows the modulation of the properties of the obtained products since a wide selection of esterifying agents can be used. The different possible combinations of the esterifying agents make possible to obtain a product with precisely selected branching degrees and, therefore, with precisely selected properties.

Known processes were not able to yield a product with an intermediate branching degree since the substitution was symmetrical and both chains of the vicinal esters groups had to be introduced at the same time. Unlike the known processes, the present process allows to attain intermediate branching degrees by fixing one of the esters and then varying the second ester chain.

The modification of the branching degree on the fatty acid chain allows to modulate the fluid properties e.g. viscosity, pour point and cloud point. Thus, it is considered a contribution to the art the provision of a process which provides a precise control of the branching degrees of the fatty acids chains and which allows to obtain products with a higher variety of branching degrees and thus with a high variety of values for the desired properties.

Accordingly, an aspect of the present invention, relates to the provision of a process for the preparation of a composition comprising at least one ester of one or more saturated C₁₈ fatty acids and an alcohol, where the fatty acids have between 1 and 3 pairs of vicinal ester groups of formula R₁COO-, the two vicinal ester groups in each pair being different, R₁ is a radical independently selected from linear or branched (C₁-C₁₈) alkyl; and the alcohol is selected from the group consisting of a (C₁-C₁₅) linear or branched monohydric alcohol and a (C₂-C₆) polyhydric alcohol having between 2 and 4 hydroxyl groups, the process comprising:
a) epoxidizing a product comprising at least an ester of an unsaturated C₁₈ fatty acid selected from the group consisting of linoleic acid, linolenic acid, oleic acid, and mixtures thereof, and an alcohol selected from the group consisting of a (C₁-C₁₅) linear or branched monohydric alcohol and a (C₂-C₆) polyhydric alcohol having between 2 and 4 hydroxyl groups, in the presence of hydrogen peroxide; and ring opening of the epoxide obtained by reaction with a (C₁-C₁₈) linear or branched carboxylic acid to yield a mono-ol ester; and
b) reacting the compound obtained in step a) with a (C₁-C₁₈) linear or branched carboxylic acid different from the one used in step a), in the presence of a suitable esterification catalyst, e.g. tin(II) chloride, tin (II) oxalate, thereby the free hydroxyl groups are esterified.

Another aspect of the present invention relates to the provision of a composition comprising at least one ester of one or more saturated C₁₈ fatty acids and an alcohol, wherein the fatty acids have between 1 and 3 pairs of vicinal ester groups of formula R₁COO-, the two vicinal ester groups in each pair being different, R₁ is independently selected from linear or branched (C₁-C₁₈) alkyl; and the alcohol is selected from the group consisting of a (C₁-C₁₅) linear or branched monohydric alcohol and a linear or branched (C₂-C₆) polyhydric alcohol having between 2 and 4 hydroxyl groups, obtainable by the process of the invention.

Due to the similar reactivity of the two carbons forming the double bonds of the unsaturation sites of the original C₁₈ fatty acids, the acid group introduced in step a) reacts indistinctly with any of them, leaving the other carbon substituted with a hydroxyl group. Thus, esters obtained after the second esterification in step b) can contain the saturated fatty acid chains in the original unsaturation sites in any of both different patterns.

Another aspect of the invention relates to the use of the composition as defined above as base stock oil for lubricants.

Another aspect of the invention relates to the use of the composition as defined above as an additive to modulate the viscosity and/or the pour point of lubricant compositions.

Finally, another aspect of the present invention relates to lubricants comprising the compositions as defined above.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The term "percentage (%) by weight" refers to the percentage of each ingredient of the combination or composition in relation to the total weight.

The term "vicinal groups" is understood as contiguous groups generated by the substitution over the carbon atoms of the unsaturation of a fatty acid, i.e. a double C-C bond on the alkyl chain of a fatty acid.

The term "catalytic amount" is understood as the amount of catalyst needed to catalyze a specific process or step.

As mentioned above, one aspect of the invention is the process for the preparation of the composition of the invention comprising the steps of a) epoxidizing at least an ester of an unsaturated C₁₈ fatty acid selected from the group consisting of linoleic acid, linolenic acid, oleic acid, and mixtures thereof, and an alcohol selected from the group consisting of a (C-Cₗ₅) linear or branched monohydric alcohol and a (C₂-C₆) polyhydric alcohol having between 2 and 4 hydroxyl groups, in the presence of hydrogen peroxide; followed by ring opening of the epoxide obtained by reaction with a (C₁-C₁₈) linear or branched carboxylic acid; and b) reacting the compound obtained in step a) with a (C₁-C₁₈) linear or branched carboxylic acid different from the one used in step a), in the presence of a catalytic amount of a suitable esterification catalyst.

A particular embodiment of the process is shown in Scheme I.

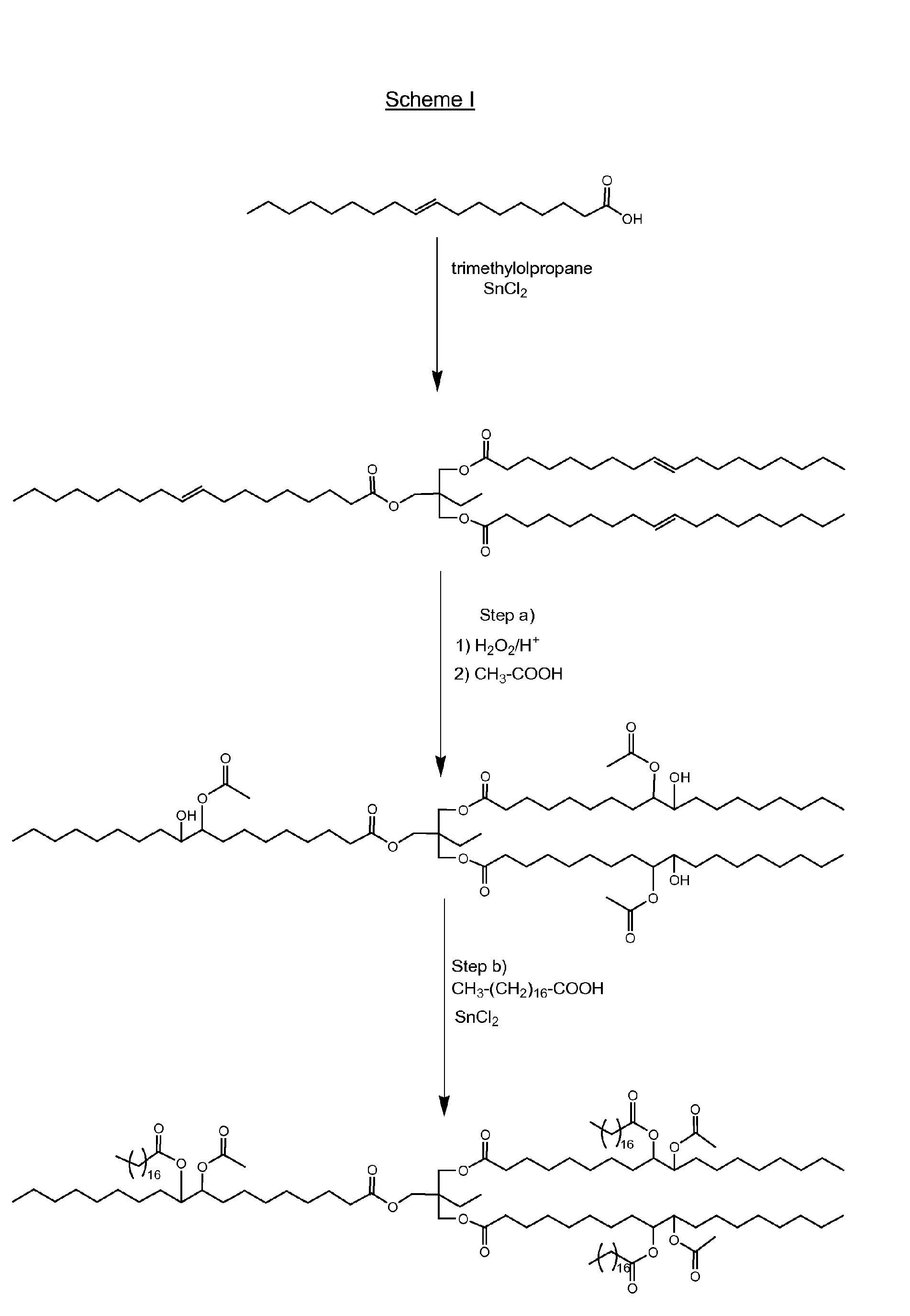

Scheme I depicts a particular embodiment of the invention wherein each fatty acid chain of the ester show the same substitution pattern on the original unsaturation sites, i.e. the acetate ester is in the farther site from the trimethylolpropane moiety of the molecule. As described above, in a general case, the order of the vicinal ester substituents could be different in each chain.

Generally, the product comprising at least an ester of an unsaturated C₁₈ fatty acid used in step a) of the process of the invention as starting material is commercially available in the form of chemical reagents or as side products of industrial processes, e.g. biodiesel preparation. However, in the case that an specific ester were not available or it were desirable to prepare it, well known processes are known in the art for the preparation of such ester using as raw materials from naturally occurring glycerine esters of fatty acids or from other fatty acid esters of synthetic origin. For instance, the skilled man in the art know processes for the preparation of esters of unsaturated C₁₈ fatty acids and alcohols by transesterification of other fatty acid esters and a desired alcohol, and by hydrolysis of the original fatty acid ester, and esterification with the desired alcohol.

Suitable oils used as fatty acid sources for the present invention are pure unsaturated C₁₈ fatty acid esters commercially available in the form of chemical reagents or natural lipid extracts containing unsaturated C₁₈ fatty acid esters found in e.g. natural oils.

Accordingly, the ester of the C₁₈ fatty acid used in the process of the invention can be a part of a natural lipid extract. Natural lipid extracts are lipid materials extracted from plants, mostly from plant seeds, including genetically modified or hybrid plants, or animals. These extracts are rich in natural unsaturated fatty acids in the form of glycerine esters. When the natural lipid extracts are processed for making them suitable for the desired application, lipid materials are usually modified by transesterification with a desired alcohol or by hydrolysis, and esterification with a desired alcohol, rendering the esters of the desired alcohols with the fatty acids naturally comprised in the natural lipid extract.

Suitable natural lipid extracts are those with a significative total content in unsaturated C₁₈ fatty acids, e.g. more than 10%, 30%, 40%, 50%, 60%, 70%, 80%, or 90% by weight. Examples of suitable natural lipid extracts with a significative total content in unsaturated C₁₈ fatty acids are vegetal oils from, without being a limitation, acai, algae, almond, amaranth, apricot, argan, artichoke, avocado, beech, brazil, cashew, castor, chia, corn, cottonseed, filbert, flax, grape, grape seed, hemp, hickory, honge, jatropha, kukui, macadamia, neem, palm, palm kernel, peanut, pecan, pistachio, evening primrose, olive, olive kernel, pumpkin, rape (canola), radish, ramtil, rice bran, safflower, salicornia, sesame, soybean, sunflower, tigernut, walnut, wheat germ, and wood pulp (tall oil) or from animal origin such as tallow. Preferably, natural oils with a significative total content in unsaturated C₁₈ fatty acids are extracted from olive kernel, palm, palm kernel, sunflower, sunflower seed, wood pulp (tall oil), and tallow. The most preferred natural lipid extract for the use in the present invention is olive kernel oil.

The following Table 1 (see, F.D. Gunstone et al., The Lipid Handbook, Chapman & Hall (London), 2nd Edition, 1994, pp. 118-119) shows the content in unsaturated C18 fatty acids of selected natural extracts.

**Table 1:**

| Unsaturated C₁₈ fatty acid percentage in natural extracts (w/w) | | | |
|---|---|---|---|
| Origin | Linolenic acid (%) | Linoleic acid (%) | Oleic Acid (%) |
| Cottonseed | 0.0-0.04 | 46.7-58.2 | 14.7-21.7 |
| Grape seed | 0.0-0.1 | 58-78 | 12-28 |
| Olive | <1.5 | 3.5-21 | 55-83 |
| Palm | 0.0-0.5 | 6.5-12.0 | 36.0-44.0 |
| Palm kernel | - | 1.0-3.5 | 12.0-19.0 |
| Sunflower seed | 0.0-0.2 | 48.3-74.0 | 14.0-39.4 |
| Tallow | <2.5 | 0.5-5.0 | 26-50 |

The process of the invention can comprise a further previous step wherein an ester of unsaturated C₁₈ fatty acids, a mixture thereof, or a natural lipid extract comprising at least one ester of unsaturated C₁₈ fatty acid, e.g. an unsaturated C₁₈ triglyceride, the fatty acid being selected from the group consisting of linoleic acid, linolenic acid, oleic acid, and mixtures thereof is transesterified with a desired alcohol, or first is hydrolyzed and then esterified with the desired alcohol. The desired alcohols are selected from the group consisting of a (C₁-C₁₅) linear or branched monohydric alcohol and a (C₂-C₆) linear or branched polyhydric alcohol having from 2 to 4 hydroxyl groups, in the presence of a catalyst, to yield at least an ester of the selected alcohol and the selected unsaturated C₁₈ fatty acid.

Any esterification catalysts, which are well-known by the skilled man in the art, can be used in the previous esterification step. For instance, suitable catalysts are Lewis acids, Lewis bases, metal salts, metal hydrides, metal halides, and ion exchange resins. Preferably, the catalyst is a salt, hydride or halide of a metal selected from sodium, magnesium, tin and zinc. More preferably, the catalyst is a tin halide. The most preferred tin halide is tin dichloride. Generally, catalyst is added in an amount from 0.1 to 2 % by weight.

Preferably, the previous esterification step is carried out in the presence of SnCl₂ as catalyst under inert atmosphere for 24 h at 200 °C.

In a preferred embodiment of the present invention, the C₁₈ fatty acid is selected from the group consisting of linoleic acid and oleic acid. In a more preferred embodiment, the C₁₈ fatty acid is oleic acid.

The alcohols used in the esterification step and forming part of the starting ester material of step a) can be selected from monohydric and polyhydric alcohols. In a preferred embodiment, the alcohol is a (C₄-C₆) linear or branched polyhydric alcohol, having from 2 to 4 hydroxyl groups. Preferably, the (C₄-C₆) polyhydric alcohol is selected from the group consisting of pentaerythritol, neopentilglycol, and trimethylolpropane. More preferably, the (C₄-C₆) polyhydric alcohol is trimethylolpropane.

In another preferred embodiment, the alcohol is a monohydric alcohol. Preferably, the monohydric alcohol is selected from the group consisting of methanol, ethanol, propanol, isopropanol, butanol, hexanol, 2-ethyl hexanol, octanol, isodecanol and isotridecanol. More preferably, the monohydric alcohol is selected from the group consisting of methanol, isopropanol, 2-ethyl hexanol, isodecanol, and isotridecanol.

The selection of the (C₁-C₁₈) linear or branched carboxylic acid used in step a) and of the one used in step b) of the process of the present invention provides control of the branching degree of the final product. As stated before, the process of the invention provides means for improving the modulation of the properties of the range of products obtained. The introduction of the different possible combinations of asymmetrical vicinal esters in the unsaturation sites of a fatty acid ester by selecting different (C₁-C₁₈) linear or branched carboxylic acids in step a) and in step b) make possible to obtain a product with precisely selected branching degrees, thus providing a precise control over the properties of the final product, e.g. viscosity, pour point and cloud point, and providing a range of products with very wide intervals of these properties.

In a particular embodiment of the invention the (C₁-C₁₈) carboxylic acid of step a) is selected from the group consisting in formic acid, acetic acid, propionic acid, butyric acid, and hexanoic acid.

In a preferred embodiment of the invention, the (C₁-C₁₈) linear or branched carboxylic acid of step a) is acetic acid.

In another preferred embodiment of the invention, the (C₁-C₁₈) linear or branched carboxylic acid of step b) is a (C₁-C₁₄) linear or branched carboxylic acid. In a more preferred embodiment, the (C₁-C₁₈) linear or branched carboxylic acid of step b) is (C₅-C₁₄) linear or branched carboxylic acid. Examples of suitable (C₁-C₁₈) linear or branched carboxylic acids for the purposes of the invention are formic acid, acetic acid, propionic acid, butyric acid, hexanoic acid, 2-ethylhexanoic acid, octanoic acid, nonanoic acid, decanoic acid, dodecanoic acid, tetradecanoic acid, hexadecanoic acid, and octadecanoic acid.

The different steps of the process of the invention do not require the use of any solvent provided that the starting materials are liquid and can be reacted simply by contacting them pouring one over the other. However, suitable organic solvents can be used to adjust the viscosity of the reaction mixture to optimize the process to particular starting materials.

The selection of the reaction conditions of step a) allows performing optimally the epoxidation and the ring opening over the unsaturations on the fatty acid chains. In a preferred embodiment of the invention the epoxidation reaction of step a) is carried out at a temperature comprised between 50 and 90 °C. In a more preferred embodiment, step a) is carried out at a temperature of about 75°C. Generally, the reaction time is comprised between 3 and 8 hours, although it can depend on the working scale. Also generally, the reaction is carried out with an excess of (C₁-C₁₈) acid, preferably between 1.1 to 3 molar excess relative to moles of unsaturation of the starting compound.

Unlike some processes known in the art, the process of the invention avoids the formation of the diol compound resulting from the opening of the intermediate epoxy ring by reaction with water. The present process allows the opening of the epoxy ring with the (C₁-C₁₈) carboxylic acid present in the reaction mixture, avoiding the formation of a diol intermediate. In the present invention, all reactants of the ring opening can be added initially to the reaction mixture and epoxide formation and ring opening is produced simultaneously or sequentially in the same reaction. Thus, besides obtaining unique asymmetrically substituted ester products, the efficiency, reliability and yield of the reaction is improved with regard to known epoxidation-ring opening processes disclosed in the art.

In a particular embodiment, the invention provides a process wherein the esterification in step b) which is carried out by reaction of a (C₁-C₁₈) linear or branched carboxylic acid different from the one used in step a), in the presence of a catalytic amount of a metal catalyst is carried out at a temperature comprised between 150 and 250 °C. Generally, the reaction time is comprised between 1 and 7 days.

Any esterification catalysts, which are well-known by the skilled man in the art, can be used in step b). For instance, suitable catalysts are Lewis acids, Lewis bases, metal salts, metal hydrides, metal halides, and ion exchange resins. Preferably, the catalyst is a salt, hydride or halide of a metal selected from sodium, magnesium, tin and zinc. More preferably, the catalyst is a tin halide. The most preferred tin halide is tin dichloride. Generally, tin dichloride is added in an amount from 0.1 to 2 % by weight.

The product obtainable through the process of the invention is a composition comprising at least one ester of one or more saturated C₁₈ fatty acids and an alcohol, where the fatty acids have between 1 and 3 pairs of vicinal ester groups of formula R₁COO-, the two vicinal ester groups in each pair being different, R₁ is a radical independently selected from linear or branched (C₁-C₁₈) alkyl; and the alcohol is selected from the group consisting of a (C₁-C₁₅) linear or branched monohydric alcohol and a (C₂-C₆) polyhydric alcohol having between 2 and 4 hydroxyl groups.

Some specific examples of the esters which can be comprised in the compositions obtainable by the process of the present invention are esters of oleic acid and trimethylolpropane, with 1 pair of vicinal ester groups per fatty acid chain, where one is acetate and the other is as follows: formic acid, acetic acid, propionic acid, butyric acid, hexanoic acid, 2-ethylhexanoic acid, octanoic acid, nonanoic acid, decanoic acid, dodecanoic acid, tetradecanoic acid, hexadecanoic acid, and octadecanoic acid.

When the starting material of the process of the invention is a natural lipid extract, the composition obtainable by the process of the present invention comprises mixtures of various esters of the different saturated fatty acids corresponding to those unsaturated fatty acids contained in the natural lipid extract.

The compositions of the invention show properties that make them useful as lubricants, in particular as lubricants base stock oils and as additives to modulate the viscosity of lubricant compounds.

Example 5 illustrates in comparative way some common values of the known prior art compounds and the compounds of the invention. From the comparative data, it reveals that differences between an ether bond and an ester bond and a symmetrical and asymmetrical substitution of the unsaturation sites are very important in the properties of the final composition. One of the major difference between the compositions of the invention and the products known in the art is that the compositions of the invention allow to obtain a viscosity range which is wider and superior to any of the biolubricants known, while maintain good properties in cold and a good viscosity index as it is illustrated in Example 4.

As mentioned above, the composition of the present invention can be used as lubricant base stock oil. It can also be used as an additive to modulate the viscosity of lubricant compounds and as a pour point depressant. Natural and synthetic base stock oils may be used alone or blended and formulated into a lubricating composition. In a lubricant composition the base stock oil is the major constituent and contributes significantly to their properties. In general, a few base oils are used to manufacture a wide variety of lubricant compositions by varying the mixtures of individual base oils and individual additives.

It also forms part of the invention a lubricant composition comprising the composition of the invention.

The lubricants comprising the composition of the invention can be formulated with one or more additives to its use as cost effective, high performance, and readily biodegradable industrial oils, such as high performance hydraulic fluids or engine and anti-wear lubricants. Typically, additives are present in lubricants ranging from about 0.1 % to about 20% based on weight. The additives which may be included in the compositions of the present invention include soluble extreme-pressure and anti-wear agents, oxidation and thermal-stability improvers, corrosion-inhibitors, viscosity index improvers, pour point and/or floc point depressants, detergents, dispersants, anti-foaming agents, viscosity adjusters, etc. For example, an engine transmission fluid can be formulated comprising antioxidants; anti-foam additives, antiwear additives, corrosion inhibitors, dispersants, detergents, and acid neutralizers, or combinations thereof. Hydraulic oil formulations can include antioxidants, anti-rust additives, anti-wear additives, pour point depressants, viscosity index improvers and anti-foam additives or combinations thereof. Specific oil formulations will vary depending on the end use of the oil; suitability of a specific formulation for a particular use can be assessed using standard techniques.

Viscosity index can be increased by adding viscosity modifiers such as polyisobutylenes, polymethacrylates, polyacrylates, vinyl acetates, ethylene propylene copolymers, styrene isoprene copolymers, styrene butadiene copolymers, or styrene maleic ester copolymers.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

The characterization of the physical properties of the products has been done following the ASTM standards.

Kinematic viscosity (indistinctly mm²/s or cSt which are equivalent) has been measured at 40°C and 100°C according to ASTM Standard D ― 445. ASTM method D ― 445 specifies a procedure for the determination of the kinematic viscosity of liquid products at specified temperature, by measuring the time for a volume of liquid to flow under gravity through a calibrated glass capillary viscometer. The kinematic viscosity is of key importance in lubrication as the correct operation of the equipment depends upon the appropriate viscosity of the liquid being used.

Viscosity Index has been obtained according to ASTM Standard D - 2270. ASTM method D - 2270 specifies the procedures for calculating the viscosity index of lubricating oils from their kinematic viscosities at 40°C and 100 °C. The viscosity index is a widely used and accepted measure of the variation in kinematic viscosity due to changes in the temperature and in practice is expressed as a single number, where a higher viscosity index indicates a smaller decrease in kinematic viscosity with increasing temperature of the lubricant.

Pour point (°C) has been measured according to ASTM Standard ― D 97. ASTM method D ― 97 specifies a procedure for the determination of the pour point of liquid products. After a preliminary heating, the sample is cooled at a specified rate and examined at intervals of 3 °C for flow characteristics and the lowest temperature at which movement of the specimen is observed is recorded. The pour point of lubricating oils is an index of the lowest temperature of its utility for certain applications.

Flash point (°C) has been measured according to ASTM Standard - 92. ASTM method D ― 92 describes the determination of the flash point and fire point of petroleum products by a Cleveland open cup apparatus. Approximately 70 ml of test specimen is filled into a test cup. The temperature of the test specimen is increased rapidly at first and then at a slower constant rate as the flash point is approached. At specified intervals a test flame is passed across the cup. The flash point is the lowest liquid temperature at which application of the test flame causes the vapours of the test specimen of the sample to ignite. The flash point is one measure of the tendency of the test specimen to form a flammable mixture with air under controlled laboratory conditions.

All the titrations have been done following the official A.C.O.S methods.

Acid value expresses the weight in mg of potassium hydroxide required to neutralize one gram of fatty material. The acid value is reported as "mg KOH / g sample".

Hydroxyl value is the number of mg of potassium hydroxide required to neutralize the acetic acid capable of combining by acetylation with one gram of fat. The hydroxyl value is reported as "mg KOH / g sample".

Iodine value of a fat is a measure of its degree of unsaturation and is conventionally expressed as the weight of iodine absorbed by 100 parts by weight of the fat. The iodine value is reported as "g l₂ / 100 g sample".

The saponification value denotes the weight in mg of potassium hydroxide required to saponify one gram of fat. The saponification value is reported as "mg KOH / g sample".

The oxirane oxygen value is expressed as the percentage of oxygen contained in the oxirane rings of a fatty material. The oxirane oxygen value is reported as "g O/100 g sample".

### Example 1: Preparation of trimethylolpropane tri-ester of a mixture of C₁₈ unsaturated fatty acid

The process was carried out in a 2 L glass jacketed reactor. The reactor was further equipped with a vacuum distillation head, thermostatic bath, stirring and nitrogen flow.

The starting fatty acid (OA) was a mixture of oleic (65-75%), linoleic (4-14%), and linolenic (<18%) acids and other fatty acids as myristic, palmitic, and stearic acid, having an acid value of 198.6 mg KOH / g sample and a iodine value of 76.6 g l₂ /100 g sample. The starting alcohol was trimethylolpropane (TMP), with a hydroxyl value of 1346 mg KOH / g sample.

Initially, 664.4 g of the mixture of fatty acids (OA) was added to the reactor together with 104.6 g of the alcohol (TMP) and 0.75 g of SnCl₂ (1 % by weight). The initial mixture showed a slight excess of 10 mg KOH/g sample in hydroxyl value to obtain a final ester with a low acid value. The mixture was heated to 200°C under N₂ and stirred. After 24 h, the reactor was cooled down to 75°C and then the reaction mixture was washed twice with deionized water to remove the catalyst, the volume of water added in each wash was the third part of the volume of oil in the reactor. Finally, the remaining water was distilled off to yield 750 g of trimethylolpropane tri-ester (OTMP).

**Table 2 ― Titration values for the esterification reaction to obtain OTMP**

| **Assay** | **Initial value** | **Final Value** | **Units** |
|---|---|---|---|
| Acid Value | 170.63 | 0.5 | mg KOH / g sample |
| Hydroxyl Value | 183.53 | 12.2 | mg KOH / g sample |
| Saponification Value | 0.00 | 170.1 | mg KOH / g sample |

Table 2 shows the titration values obtained for the esterification reaction. With these values it is possible to calculate the mol present in 750 g of OTMP, which allows for conversion calculations. An esterification reaction can be monitored by the acid or hydroxyl value decrease or the saponification value increase, where more reliable results are obtained by the hydroxyl value.

**Table 3 ― Conversion for the esterification reaction to obtain OTMP**

| **Initial Product** | **Final Product** | **Group** | **Initial mol** | **Final mol** | **C (%)** |
|---|---|---|---|---|---|
| **OA + TMP** | **OTMP** | Hydroxyl | 2.54 | 0.163 | 94% |

Table 3 shows the quantitative conversion (94%) according to the hydroxyl groups decrease to form ester groups under these reaction conditions.

### Example 2: Preparation of the trimethylolpropane tri-ester with two vicinal hydroxyl groups, one being a free OH and the other esterified to give the acetate by a one pot epoxidation-ring opening process of the trimethylolpropane tri-ester of the mixture of C₁₈unsaturated fatty acids of Example 1

Regarding the unsaturation mol obtained by the iodine value of the product obtained in the previous step (OTMP), stoichiometric amounts of reagents were added. For 750 g OTMP the following reagents, acetic acid (2.2 mol/mol unsat., 4.25 mol, 255 g), hydrogen peroxide (3 mol/mol unsat., 5.79 mol, 600 ml 30% H₂O₂ solution) and sulphuric acid (2 % by weight of the volume of acetic acid and hydrogen peroxide used) were added. The acetic acid was added to the crude obtained in the previous step (OTMP) at room temperature and the reactor was heated up to 75°C. Next, 10 ml H₂SO₄ were added and finally 600 ml H₂O₂ 30% was added dropwise during 1 hour. After 5 hours the reaction was completed and the trimethylolpropane tri-ester mono-ol acetate (OH_C2) was obtained.

Finally, the reaction mixture was neutralized to stop the reaction. While maintaining the temperature and stirring of the reactor, it was further dropwise added a solution of 80 ml of 20% NaOH, the amount required to neutralize the sulfuric acid. Once all the NaOH was added stirring was stopped and the two phases were let to separate (rapid separation) and the aqueous phase was discarded. The organic phase was washed again with 250 ml of a saturated solution of NaHCO₃ (70 g / L). The separated aqueous phase showed a pH of about 7. Then the organic phase was washed twice with 250 ml of water. The small amount of water forming an emulsion with the organic phase was distilled off. The product obtained was 850g of the trimethylolpropane tri-ester mono-ol acetate (OH_C2), anhydrous and free of mineral acid.

**Table 4 ― Titration values for the epoxidation ― ring opening reaction to obtain OH_C2**

| **Assay** | **Initial value** | **Final Value** | **Units** |
|---|---|---|---|
| Acid Value | 0.5 | 17.0 | mg KOH / g sample |
| Hydroxyl Value | 12.2 | 170.0 | mg KOH / g sample |
| Iodine value | 76.60 | 0.6 | g l₂/100 g sample |
| Oxirane Oxygen | 0.00 | 0.00 | g O/100 g sample |

Table 4 shows the titration values obtained for the epoxidation ― ring opening reaction. With these values it is possible to calculate the mol present in 850 g of OH_C2, which allows for conversion calculations. Epoxidation reactions are monitored by the decrease of iodine value and their selectivity is determined by the comparison of the real oxirane oxygen value with the theoretical maximum value obtained by the initial iodine value (as one oxirane ring is obtainable for each unsaturation). The ring opening is monitored by comparing the real hydroxyl value with the theoretical maximum value obtained by the initial iodine value (as 2 hydroxyl groups are obtainable for each unsaturation). It is also possible to monitor the ester group hydrolysis by comparing the acid value with the saponification value of the starting material.

**Table 5 ― Conversion for the epoxidation ― ring opening reaction to obtain OH_C2**

| **Initial Product** | **Final Product** | **Group** | **Initial mol** | **final mol** | **C (%)** |
|---|---|---|---|---|---|
| **OTMP** | **OH_C2** | Acid | 0.007 | 0.258 | 13% |
| | | Unsaturation | 1.93 | 0.017 | 98% |
| | | Oxirane | 0.00 | 0.00 | 0% |
| | | Hydroxyl | 0.163 | 2.576 | 41% |

Final OH_C2 product comprised 13% of free acid due to the hydrolysis of OTMP occurring during the epoxidation reaction. The unsaturation of the starting product (OTMP) was reduced by 98% showing that the OH_C2 product obtained was almost saturated. Oxirane oxygen value showed no presence of epoxides indicating its full opening. As the unsaturation had disappeared, the hydroxyl value indicates that almost all oxirane rings have been converted to mono-ol acetate (41 % out of 50% theoretical value).

### Example 3: Preparation of trimethylolpropane tri-ester with two vicinal hydroxyl groups, one being esterified as acetate and the other as an alkanoate C6, C8, C10, C12, C14, or C16 (C2 CX, X =6, 8, 10, 12, 14, or 16)

To 850 g of trimethylolpropane tri-ester mono-ol acetate (OH_C2), were added 611.1 g of stearic acid. The initial mixture showed a slight excess of 10 mg KOH/g sample in hydroxyl value to obtain an ester with a low acid value. The mixture was heated to 200 ° C under nitrogen atmosphere. Once the temperature was reached, 1.461 g of SnCl₂ (1 % by weight) were added and allowed to react for 5 days to afford 1450 g of trimethylolpropane tri-ester stearate-acetate (C18_C2). The crude reaction was washed twice with 500 ml of deionized water to remove the catalyst and distilled off to yield the final product.

**Table 6 ― Titration values for the esterification reaction to obtain C18_C2**

| **Assay** | **Initial value** | **Final Value** | **Units** |
|---|---|---|---|
| Acid Value | 97.44 | 1.5 | mg KOH / g sample |
| Hydroxyl Value | 111.83 | 9 | mg KOH / g sample |

Table 6 shows the titration values obtained for the esterification reaction. With these values it is possible to calculate the mol present in 1450 g of C18_C2, which allows for conversion calculations. An esterification reaction can be monitored by the acid or hydroxyl value decrease or the saponification value increase, where more reliable results are obtained by the hydroxyl value.

**Table 7 ― Conversion for the esterification reaction to obtain C18_C2**

| **Initial Product** | **Final Product** | **Group** | **initial mol** | **final mol** | **C (%)** |
|---|---|---|---|---|---|
| **OH_C2** | **C18_C2** | Hydroxyl | 2.89 | 0.23 | 92% |

Table 7 shows the conversion (92%) according to the hydroxyl groups decrease to form ester groups under these reaction conditions.

Table 8 shows the reaction conditions used to generate the family of branched compounds, which have a side chain of 6 to 18 carbons. Different batches of the product OH_C2 have been used (with the same product characteristics). A different fatty acid in each product has been used, in order to have the desired length for branching. A slight excess of 10 mg KOH/g sample in hydroxyl value was always used in order to obtain an ester with a low acid value. The reaction temperature was always 200 °C, unless this is greater than the boiling point of fatty acid used as in the case of product C2_C6. The catalyst used in all cases has been the SnCl₂. The reaction time has been a little variable as it was fundamental to obtain products with high degree of branching (close to 90%) and a low final acid value (below 2 mg KOH / g sample).

**Table 8:**

| **Product** | **Alcohol** | **Acid** | **T (°C)** | **t (h)** | **C (%)** |
|---|---|---|---|---|---|
| C2_C6 | C2_OH | Hexanoic | 170 | 200 | 87% |
| C2_C8 | C2_OH | Octanoic | 200 | 113 | 83% |
| C2_C10 | C2_OH | Decanoic | 200 | 115 | 85% |
| C2_C12 | C2_OH | Capric | 200 | 71 | 85% |
| C2_C14 | C2_OH | Myristic | 200 | 91 | 90% |
| C2_C16 | C2_OH | Palmitic | 200 | 144 | 90% |

In the previous Table 8, C means conversion from hydroxyl groups.

### Example 4: Evaluation of the properties of the compositions of the invention

The compounds evaluated are esters of trimethylolpropane and C₁₈ acid fatty chains modified from oleic acid to include pairs of vicinal ester groups in the sites wherein oleic acid was unsaturated, where one of the ester groups in a pair is acetate and the vicinal ester group is as follows: hexanoate (compound C2_C6), octanoate (compound C2_C8), decanoate (compound C2_C10), laurate (compound C2_C12), myristate (compound C2_C14), palmitate (compound C2_C16), or stearate (compound C2_C18).

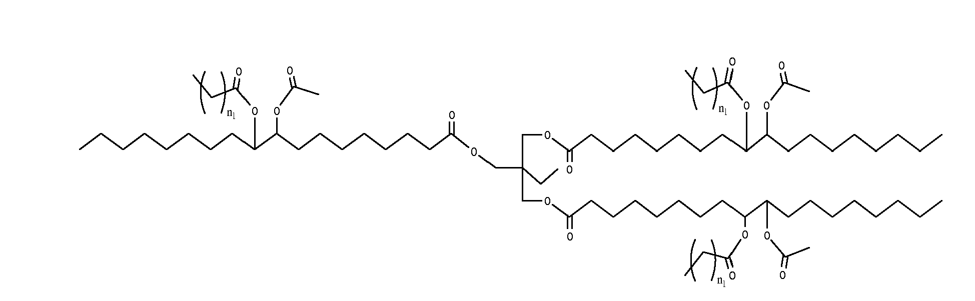

For comparison, compound OH_C2 corresponds to an ester of trimethylolpropane and C₁₈ acid fatty chains modified from oleic acid to include OH groups on the original unsaturation sites, where only one of them is esterified with acetate. The results obtained are included in Table 4.

**Table 4:**

| **Compound** | **Pour point (°C)** | **Viscosity 40 °C (cSt)** | **Viscosity index (V.l.)** | **Flash Point (°C)** |
|---|---|---|---|---|
| C2_C6 | -6 | 1049.9 | 160 | 298 |
| C2_C8 | -3 | 801.4 | 158 | 290 |
| C2_C10 | -9 | 798.6 | 164 | 302 |
| C2_C12 | -9 | 753.6 | 165 | 298 |
| C2_C14 | 0 | 679.6 | 169 | 280 |
| C2_C16 | 9 | 742.9 | 168 | 298 |
| C2-C18 | 27 | 888.1 | 156 | 300 |
| OH_C2 | 9* | 1644.0 | 96 | 312 |

| | | | | |
|---|---|---|---|---|
| *The compound is a solid at room temperature, but the pour point measured according to the ASTM method D-97 is 9°C. | | | | |

The results of the previous Table 4 show the positive effect of esterifying both hydroxyl groups on the properties in cold. Moreover, the viscosity is reduced and the viscosity index is improved. As it is derived from Table 4, the viscosity can be modulated according to these Examples from 680 cSt to 1050 cSt. As it is mentioned in the description the compounds of the invention show a high oxidative stability due to the absence of unsaturations, but maintaining good properties in cold.

### Comparative Example 5: Comparison of the properties of the compositions of the invention with those of the known prior art lubricant compounds

The structural differences of different types of esters of saturated fatty acids involve different final properties as lubricants. The properties of several groups of known compounds have been compared with the compositions of the invention.

Type la: Ester of a saturated C₁₈ fatty acid and a (C₁-C₁₈) alcohol with a pair of vicinal hydroxyl groups, one hydroxyl group being in free form and the other etherified as a (C₁-C₁₈) alkoxy of equal length than the alcohol of the fatty acid ester.

Type lb: Ester of a saturated C₁₈ fatty acid and a (C₁-C₁₈) alcohol with a pair of vicinal hydroxyl groups, one hydroxyl group etherified as a (C₁-C₁₈) alkoxy of equal length than the alcohol of the fatty acid and the other hydroxyl group esterified as a (C₁-C₁₈) alkoxy of different length than the alcohol of the fatty acid ester.

Type lc: Ester of a saturated C₁₈ fatty acid and a (C₁-C₁₈) alcohol with a pair of vicinal hydroxyl groups, one hydroxyl group being in free form and the other hydroxyl group being etherified as a (C₁-C₁₈) alkoxy of different length than the alcohol of the fatty acid ester.

Type ld: Ester of a saturated C₁₈ fatty acid and a (C₁-C₁₈) alcohol with a pair of vicinal hydroxyl groups, one hydroxyl group etherified as a (C₁-C₁₈) alkoxy of different length than the alcohol of the fatty acid ester, the other hydroxyl group being esterified with a (C₁-C₁₈) carboxylic acids of different length than the alcohol of the fatty acid ester and of the ether.

Type II: Ester of a saturated C₁₈ fatty acid and a (C₁-C₁₈) alcohol with a pair of vicinal hydroxyl groups, both hydroxyl groups being equally esterified with a (C₁-C₁₈) carboxylic acids but of different length than the alcohol of the fatty acid ester

Type III: Ester of a saturated C₁₈ fatty acid and a (C₁-C₁₈) alcohol with a pair of vicinal hydroxyl groups, being one hydroxyl group in free form and the other esterified with a (C₁-C₁₈) carboxyl acid of different length than the alcohol of the fatty acid

In the following Table 5 the range of values obtained or found in the literature for several properties of some compounds of each type have been included in order to compare the properties of these compounds with the ones of the compounds of the invention. Cf. Hwang et al., "Modification of epoxidized soybean oil for lubricant formulations with improved oxidative stability and low pour point", JAOCS, 2001, vol. 78, n. 12, pp. 1179 - 1184; Hwang H. et al., "Preparation and Properties of Lubricant Basestocks from Epoxidized Soybean Oil and 2-Ethylhexanol" JAOCS, 2003, Vol. 80, no. 8, pp. 811- 815; Hwang H. et al. "Synthetic Lubricant Basestocks from Epoxidized Soybean Oil and Guerbert Alcohols" Industrial Crops and Products, 2006, Vol. 23, pp. 311 - 317; Lathi P. S. et al., "Green Approach for the Preparation of Biodegradable Lubricant Basestock from Epoxidized Vegetable Oil" Applied Catalysis B: Environmental, 2007, Vol. 69, pp. 207 ― 212; Erhan et al., "Chemical modification of vegetable oils for lubricant base stocks" 2003 Spring Technical Conference of the ASME Internal Combustion Engine Division, American Society of Mechanical Engineers, Salzburg, Austria, pp. 369-380; Sharma B. K. et al. "Ester Hydroxy Derivatives of Methyl Oleate: Tribological, Oxidation and Low Temperature Properties" Bioresource Technology, 2008, Vol. 99, pp. 7333 ― 7340; Salimon J. et al. "Oleic Acid Diesters: Synthesis, Characterisation and Low Temperature Properties", European Journal of Scientific Research, 2009, Vol. 32, no. 2, pp. 216 ― 222; Erhan et al. "Lubricant base stock potential of chemically modified vegetable oils" J. Agric. Food Chem., 2008, Vol. 56, issue 19, pp. 8919-8925; Salimon J. et al., "Preparation and Characteristic of 9,10-Epoxyoleic Acid α-Hydroxy Ester Derivatives as Biolubricant Base Oil", European Journal of Scientific Research, 2009, vol. 31, no. 2, 265 ― 272; Salimon J. et al. "Substituted Esters of Octadecanoic Acid as Potential Biolubricants", European Journal of Scientific Research, 2009, vol. 31, no. 2, 273 ― 279; Salimon J. et al. "Improved Low Temperature Properties of 2-Ethylhexyl-9(10)-Hyrdoxy-10(9)-Acyloxystearate Derivatives", European Journal of Scientific Research, 2009, vol. 31, no. 4, 583 ― 591.

**Table 5:**

| Compound | Pour point (°C) | Viscosity (cSt) | Viscosity index | Flash point (°C) |
|---|---|---|---|---|
| Type Ia | -36<mp<9 | 54<visc<75 | 96<IV<122 | nd |
| Type Ib | -42<mp<0 | 54<visc<75 | 127<IV<159 | nd |
| Type Ic | -18<mp<-3 | 172<visc<313 | 86<IV<113 | nd |
| Type Id | -33<mp<-3 | 74<visc<103 | 136<IV<152 | nd |
| Type I | -21 <mp<-3 | nd | nd | nd |
| Type III | -60<mp<-5 | 21<visc<290 | nd | 115<fop<197 |
| Compositions of the invention | -9<mp<27 | 680<visc<1050 | 156<IV<169 | 280<fop<312 |

| | | | | |
|---|---|---|---|---|
| nd means data non available. | | | | |

Among the improved properties of the compositions of the present invention the following can be mentioned: the viscosity range is superior to the other type of lubricant compounds, they have an exceptional viscosity index, good inflammation properties, properties in cold similar to the corresponding unsaturated compounds, and in addition they have a better biodegradability due to the ester bond.

### REFERENCES CITED IN THE APPLICATION

- WO2008/079901
- F.D. Gunstone et al., The Lipid Handbook, Chapman & Hall (London), 2nd Edition, 1994, pp. 118-119.
- Hwang et al., "Modification of epoxidized soybean oil for lubricant formulations with improved oxidative stability and low pour point", JAOCS, 2001, vol. 78, n. 12, pp. 1179-1184.
- Erhan et al., "Chemical modification of vegetable oils for lubricant base stocks" 2003 Spring Technical Conference of the ASME Internal Combustion Engine Division, American Society of Mechanical Engineers, Salzburg, Austria, pp. 369-380.
- Erhan et al., "Lubricant base stock potential of chemically modified vegetable oils" J. Agric. Food Chem., 2008, vol. 56, issue 19, pp. 8919-8925.
- Hwang H. et al., "Preparation and Properties of Lubricant Basestocks from Epoxidized Soybean Oil and 2-Ethylhexanol", JAOCS, 2003, vol. 80, no. 8, pp. 811-815.
- Hwang H. et al., "Synthetic Lubricant Basestocks from Epoxidized Soybean Oil and Guerbert Alcohols" Industrial Crops and Products, 2006, vol. 23, pp. 311-317.
- Lathi P. S. et al., "Green Approach for the Preparation of Biodegradable Lubricant Basestock from Epoxidized Vegetable Oil" Applied Catalysis B: Environmental, 2007, vol. 69, pp. 207-212.
- Sharma B. K. et al., "Ester Hydroxy Derivatives of Methyl Oleate: Tribological, Oxidation and Low Temperature Properties" Bioresource Technology, 2008, vol. 99, pp. 7333-7340.
- Salimon J. et al., "Oleic Acid Diesters: Synthesis, Characterisation and Low Temperature Properties" European Journal of Scientific Research, 2009, vol. 32, no. 2, pp. 216-222.
- Salimon J. et al., "Preparation and Characteristic of 9,10-Epoxyoleic Acid α-Hydroxy Ester Derivatives as Biolubricant Base Oil", European Journal of Scientific Research, 2009, vol. 31, no. 2, pp. 265-272.
- Salimon J. et al., "Substituted Esters of Octadecanoic Acid as Potential Biolubricants", European Journal of Scientific Research, 2009, vol. 31, no. 2, pp. 273-279.
- Salimon J. et al., "Improved Low Temperature Properties of 2-Ethylhexyl-9(10)-Hyrdoxy-10(9)-Acyloxystearate Derivatives", European Journal of Scientific Research, 2009, vol. 31, no. 4, pp. 583-591.

## Claims

1. A process for the preparation of a composition comprising at least one ester of one or more saturated C₁₈ fatty acids and an alcohol, wherein the fatty acids have between 1 and 3 pairs of vicinal ester groups of formula R₁COO-, the two vicinal ester groups in each pair being different, R₁ is a radical independently selected from linear or branched (C₁-C₁₈) alkyl; and the alcohol is selected from the group consisting of a (C₁-C₁₅) linear or branched monohydric alcohol and a (C₂-C₆) polyhydric alcohol having between 2 and 4 hydroxyl groups, the process comprising:
a) epoxidizing a product comprising at least an ester of an unsaturated C₁₈ fatty acid selected from the group consisting of linoleic acid , linolenic acid, oleic acid, and mixtures thereof; and of an alcohol selected from the group consisting of a (C₁-C₁₅) linear or branched monohydric alcohol and a (C₂-C₆) polyhydric alcohol having between 2 and 4 hydroxyl groups, in the presence of hydrogen peroxide; and ring opening of the epoxide obtained by reaction with a (C₁-C₁₈) linear or branched carboxylic acid to yield a mono-ol ester; and
b) reacting the compound obtained in step a) with a (C₁-C₁₈) linear or branched carboxylic acid different from the one used in step a), in the presence of a catalytic amount of an esterification catalyst.

2. The process according to claim 1, wherein the C₁₈ fatty acid of the ester of an unsaturated C₁₈ fatty acid is oleic acid.

3. The process according to any of the claims 1-2, wherein the monohydric alcohol is selected from the group consisting of methanol, ethanol, propanol, isopropanol, butanol, hexanol, 2-ethyl hexanol, octanol, isodecanol and isotridecanol.

4. The process according to any of the claims 1-3, wherein the polyhydric alcohol is a (C₄-C₆) polyhydric alcohol.

5. The process according to claim 4, wherein the (C₄-C₆) polyhydric alcohol is selected from the group consisting of pentaerythritol, neopentilglycol, and trimethylolpropane.

6. The process according to any of the claims 1-5, wherein the (C₁-C₁₈) carboxylic acid of step a) is selected from the group consisting of formic, acetic, propionic, butyric, and hexanoic acid.

7. The process according to claim 6, wherein (C₁-C₁₈) carboxylic acid of step a) is acetic acid.

8. The process according to any of the claims 1-6, wherein the (C₁-C₁₈) carboxylic acid of step b) is a (C₅-C₁₄) carboxylic acid.

9. A composition comprising at least one ester of one or more saturated C₁₈ fatty acids and an alcohol, wherein the fatty acids have between 1 and 3 pairs of vicinal ester groups of formula R₁COO-, the two vicinal ester groups in each pair being different, R₁ is a radical independently selected from linear or branched (C₁-C₁₈) alkyl; and the alcohol is selected from the group consisting of a (C₁-C₁₅) linear or branched monohydric alcohol and a (C₂-C₆) polyhydric alcohol having between 2 and 4 hydroxyl groups, obtainable by the process as defined in any of the claims 1-8.

10. Use of the composition as defined in claim 9 as lubricant base stock oil.

11. Use of the composition as defined in claim 10 as an additive to modulate at least one property of a lubricant compound selected from viscosity and pour point.

12. Lubricant comprising the composition of claim 9.
